# EUROPÄISCHE PATENTSCHRIFT

(11) **EP 2 467 425 B1**
(45) Veröffentlichungstag und Bekanntmachung des Hinweises auf die Patenterteilung: **07.10.2020**
(21) Anmeldenummer: 10744579.3
(22) Anmeldetag: 17.08.2010
(51) Int. Cl.: C08L 1/32, A61K 9/00, A61L 31/04, C08B 11/20, C08B 13/00, C09K 19/38

(54) **WASSERLÖSLICHE POLYSACCHARIDETHER UND IHRE VERWENDUNG**
WATER-SOLUBLE POLYSACCHARIDE ETHERS AND THE USE THEREOF
ÉTHERS DE POLYSACCHARIDE SOLUBLES DANS L'EAU ET LEUR UTILISATION

(30) Priorität: 17.08.2009 DE 102009037514
(43) Veröffentlichungstag der Anmeldung: 27.06.2012
(73) Patentinhaber: PHARMPUR GmbH, 86343 Königsbrunn (DE)
(72) Erfinder: MENZ, Dirk-Henning, 86420 Diedorf-Lettenbach (DE); RITTER, Helmut, 42111 Wuppertal (DE); MÜLLER, Bernd, 86150 Augsburg (DE)
(74) Vertreter: Charrier Rapp & Liebau
(86) Internationale Anmeldenummer: PCT/EP2010/061971
(87) Internationale Veröffentlichungsnummer: WO 2011/020829

(56) Entgegenhaltungen:
- EP-A2- 1 440 974
- DE-A1- 2 939 782
- DE-A1- 4 130 660
- US-A- 5 422 376
- WU C ET AL: "THE SYNTHESIS AND THERMOTROPIC LIQUID CRYSTALLINE BEHAVIOR OF MESOGENIC MOIETY-LINKED ETHYL CELLULOSE" POLYMER BULLETIN, SPRINGER, HEIDELBERG, DE LNKD- DOI:10.1007/S00289-002-0003-5, Bd. 48, Nr. 1, 1. März 2002 (2002-03-01), Seiten 33-41, XP001125791 ISSN: 0170-0839
- WU C ET AL: "THE SYNTHESIS AND THERMOTROPIC BEHAVIOUR OF AN ETHYL CELLULOSE DERIVATIVE CONTAINING AZOBENZENE-BASED MESOGENIC MOIETIES" LIQUID CRYSTALS: AN INTERNATIONAL JOURNAL OF SCIENCE AND TECHNOLOGY, TAYLOR & FRANCIS, GB LNKD- DOI:10.1080/0267829031000115005, Bd. 30, Nr. 6, 1. Juni 2003 (2003-06-01), Seiten 733-737, XP001161609 ISSN: 0267-8292
- V. A. E. SHAIKH, N. N. MALDAR, S. V. LONIKAR, C. R. RAJAN, S. PONRATHNAM: "Thermotropic behavior of cholesterol-linked polysaccharides" JOURNAL OF APPLIED POLYMER SCIENCE, Bd. 70, Nr. 1, 1998, Seiten 195-201, XP002606667
- V. A. E. SHAIKH, N. N. MALDAR, S. V. LONIKAR, C. R. RAJAN, S. PONRATHNAM: "Thermotropic liquid crystalline behavior of cholesterol-linked hydroxyethyl cellulose" JOURNAL OF APPLIED POLYMER SCIENCE, Bd. 72, Nr. 6, 1999, Seiten 763-770, XP002606668
- V. A. E. SHAIKH, N. N. MALDAR, S. V. LONIKAR, C. R. RAJAN, S. PONRATHNAM: "Thermotropic behavior of lithocholic acid derivative linked hydroxyethyl cellulose" JOURNAL OF APPLIED POLYMER SCIENCE, Bd. 100, Nr. 3, 2006, Seiten 1995-2001, XP002606669
- JH KIM, SY JEONG, YD MA: "Synthesis and Characteristics of Hydroxypropyl Celluloses Containing Cholesteryl and Acryloyl Groups" POLYMER KOREA, Bd. 28, Nr. 1, 2004, Seiten 92-102, XP002606670
- HU X ET AL: "Preparation, characterization and novel photoregulated rheological properties of azobenzene functionalized cellulose derivatives and their alpha-CD complexes" POLYMER, ELSEVIER SCIENCE PUBLISHERS B.V, GB LNKD- DOI:10.1016/J.POLYMER.2004.05.072, Bd. 45, Nr. 18, 19. August 2004 (2004-08-19), Seiten 6219-6225, XP004549801 ISSN: 0032-3861
- WEIYAN WANG ET AL: "Effect of [alpha]-Cyclodextrin on the Photoisomerization of Azobenzene Functionalized Hydroxypropyl Methylcellulose in Aqueous Solution" POLYMER BULLETIN, SPRINGER, BERLIN, DE LNKD- DOI:10.1007/S00289-007-0789-2, Bd. 59, Nr. 4, 8. Juni 2007 (2007-06-08), Seiten 537-544, XP019539832 ISSN: 1436-2449
- TIANHUI HU, HELOU XIE, JIANBO XIAO, HAILIANG ZHANG AND ERQIANG CHEN: "Design, synthesis, and characterization of a combined main-chain/side-chain liquid-crystalline polymer based on ethyl cellulose" CELLULOSE, Bd. 17, Nr. 3, 13. Januar 2010 (2010-01-13), Seiten 547-558, XP002606671
- Peijie Zheng ET AL: "Photoregulated Sol-Gel Transition of Novel Azobenzene-Functionalized Hydroxypropyl Methylcellulose and Its[alpha]-Cyclodextrin Complexes", Macromolecular Rapid Communications, vol. 25, no. 5, 1 March 2004 (2004-03-01), pages 678-682, XP055289171, DE ISSN: 1022-1336, DOI: 10.1002/marc.200300123
- GALLOT B ET AL: "POLY(L-LYSINE) CONTAINING AZOBENZENE UNITS IN THE SIDE CHAINS: INFLUENCE OF THE DEGREE OF SUBSTITUTION ON LIQUID CRYSTALLINE STRUCTURE AND THERMOTROPIC BEHAVIOUR", LIQUID CRYSTALS, TAYLOR & FRANCIS, vol. 23, no. 1, 1 July 1997 (1997-07-01), pages 137-146, XP000701486, ISSN: 0267-8292, DOI: 10.1080/026782997208749
- Elena-Raluca Cioanca ET AL: "SYNTHESIS, LIQUID CRYSTALLINE PROPERTIES AND THERMAL STABILITY OF 4-(4-ALKYLOXYPHENYLAZO) BENZOIC ACIDS", Analele UniversitaNii din Bucuresti, 1 January 2010 (2010-01-01), pages 39-46, XP055578592, Retrieved from the Internet: URL:http://gw-chimie.math.unibuc.ro/anuniv ch/2010-2/AUBCh1923946.pdf
- R F T Stepto ET AL: "INTERNATIONAL UNION OF PURE AND APPLIED CHEMlSTRY MACROMOLECULAR DIVISION COMMISSION ON MACROMOLECULAR NOMENCLATURE* DEFINITIONS OF BASIC TERMS RELATING TO LOW-MOLAR-MASS AND POLYMER LIQUID CRYSTALS (IUPAC Recommendations 2001) Definitions of basic terms relating to low-molar-mass and polymer liquid", Pure Appl. Chem. Associate Member from J. Work (USA, Associate Member from J. V. Alemán (Spain J.-I. Jin (Korea, 1 January 2001 (2001-01-01), pages 845-895, XP055236459, Retrieved from the Internet: URL:http://pac.iupac.org/publications/pac/ pdf/2001/pdf/7305x0845.pdf
- Römpp: Flüssige Kristalle

## Beschreibung

Die Erfindung betrifft gelartige bis zu standfeste wässrige Zubereitungen mit viskoelastischem Fließverhalten, die Polysaccharidether mit mesogenen Seitengruppen aufweisen und deren Verwendung im menschlichen Körper, insbesondere im Rahmen ophthalmologischer Eingriffe.

Es ist seit vielen Jahren bekannt, bei ophthalmologischen Eingriffen zum Schutz von Geweben und Implantaten und zur Verminderung mechanischer Kräfte, die während der Vorbereitung und Durchführung eines operativen oder diagnostischen Eingriffs zu Schäden führen könnten, so genannte ophthalmologische Viskoelastika (OVDs) einzusetzen. Diese OVDs sind in aller Regel wässrige Polymerlösungen, die durch Einstellen eines speziellen pH-Wertes und einer speziellen Osmolarität (Einstellung sowohl des polymerabhängigen kolloidosmotischen Drucks als auch des osmotischen Drucks, der durch die enthaltenen anorganischen Salze bestimmt wird) an die Gegebenheiten des menschlichen Auges angepasst worden sind. Als Basispolymere für diese Zubereitungen sind insbesondere Hyaluronsäure bzw. deren physiologisch verträgliche Salze, Chondroitinsulfat und Hydroxypropylmethylcellulose bekannt.

Eine häufige Anwendung dieser Substanzen liegt darin, dass man bei einer Kataraktoperation, also beim Ersatz der natürlichen Augenlinse, die Augenkammer und den Kapselsack zwischen Entfernung der natürlichen Linse und der Implantation der künstlichen neuen Linse mit dem Viskoelastikum füllt, um ein Zusammenfallen dieses Hohlraumes zu vermeiden. Gleichzeitig schützen die Viskoelastika die betroffenen Gewebe und unterstützen die Gleitfähigkeit der eingesetzten Instrumente und Implantate. Im weiteren Verlauf der Operation müssen die Viskoelastika dann wieder entfernt werden.

Aufgrund dieser Gegebenheit ist es erforderlich, dass ophthalmologische Viskoelastika die folgenden Fließeigenschaften aufweisen: in Abwesenheit von Scherkräften müssen sie formstabile Gele bilden, andererseits müssen sie jedoch durch die Kanüle einer Spritze leicht injizierbar und aspirierbar sein. Ein entsprechendes Fließverhalten wird sehr gut von Lösungen der Hyaluronsäure bzw. deren Salzen angenähert. Bei der Hyaluronsäure handelt es sich um ein natürlich vorkommendes Biopolymer aus der Gruppe der Glukosaminoglukane. Obgleich die Substanz weit verbreitet ist, ist es doch kostenintensiv, ergiebige Quellen für hochmolekulare Hyaluronsäure zu erschließen. Überwiegend erfolgt dies durch zwei Verfahren, nämlich die Extraktion aus tierischen Geweben oder durch eine fermentatorische Herstellung. Die Produkte aus tierischen Quellen übertreffen die fermentatorisch erzeugten Produkte in der Regel in den vom Molekulargewicht abhängigen Eigenschaften. So sind Ausgangsstoffe von kommerziell verfügbaren Produkten mit besonders hohen Null- Scherviskositäten meist tierischen Ursprungs und zeigen ein mittleres Molekulargewicht von 4 Millionen Dalton. Die fermentatorisch hergestellten Produkte besitzen ein Molekulargewicht von ca. 2,5 Millionen Dalton und entsprechend geringere Nullscherviskositäten. Der Einsatz von Produkten tierischen Ursprungs ist mit dem Risiko einer BSE/TSE Übertragung verbunden. Außerdem erfordert ihr Einsatz ebenso wie die Verwendung von fermentatorisch hergestellten Produkten eine strenge Kontrolle des Endotoxingehalts, da Endotoxine bei der Herstellung das Produkt kontaminieren können.

Hyaluronsäuresalzlösungen werden nicht nur in der Ophthalmologie, sondern auch in der Rheumatologie und Orthopädie, z.B. zur Arthrosebehandlung, eingesetzt. Einerseits kann durch Ergänzung der Sinovialflüssigkeit die Gleitfähigkeit der Knorpel und die Absorption von schockartig einwirkenden Kräften verbessert werden, andererseits werden entzündungshemmende Wirkungen diskutiert. Auch Anwendungen in der Dermatologie und plastischen Chirurgie, z.B. zum Unterspritzen von Falten, haben große Verbreitung gefunden.

Wenngleich Hyaluronsäuresalzlösungen gute technische Eigenschaften aufweisen und außer den genannten Anwendungen weitere Einsatzgebiete in der Medizin erobert haben, besteht doch Bedarf, entsprechende Produkte aus einfacheren, leichter zugänglichen und weniger kostenintensiven Quellen zu erhalten. So wird in der internationalen Anmeldung WO2008/035372, Reliance Life Sciences Pvt. Ltd. vorgeschlagen, Hyaluronsäure aus Bakterien zu gewinnen. Diese fermentatorisch gewonnenen Produkte besitzen aber durch ihre geringere Molekülmasse im Vergleich zu Hyaluronaten aus höheren Organismen limitierte rheologische Eigenschaften. Außerdem unterliegt auch die bakteriell hergestellte Hyaluronsäure dem Hauptnachteil dieser Substanzklasse, der darin besteht, dass die wässrigen Lösungen thermisch sensibel sind und daher bei Lagerung und Transport gekühlt werden müssen. Außerdem verlieren die Präparate bei einer Dampfsterilisation stark an Viskosität durch Verringerung des Molekulargewichtes. Gerade die Viskosität in Lösung ist jedoch die entscheidende technische Eigenschaft der Produkte, so dass hier ein manifester Nachteil vorliegt.

Es hat daher nicht an Versuchen gefehlt, synthetische Polysaccharidether, wie z. B. Hydroxypropylmethylcelluose, für diese Anwendung einzusetzen. So schlägt US-Patent 5,422,376, Dow Chemical, vor, ein viskoelastisches Material auf Basis einer Hydroxypropylmethylcellulose mit einem Molekulargewicht zwischen 375000 und 420000 Dalton aufzubauen. Hydroxypropylmethylcellulose (HPMC) hat sich als hervorragend körperverträglich erwiesen und ist leicht aus Celluloserohstoffen, wie sie umfänglich in der Natur vorliegen, zu gewinnen. Nachteilig ist jedoch, dass die Viskosität wässriger Zubereitungen nicht in der Weise scherkraftabhängig ist, wie dies von der Hyaluronsäure her bekannt ist. Standfeste HPMC-Lösungen lassen sich daher nicht in einfacher Weise durch eine Spritzenkanüle dosieren. Außerdem wären zum Erreichen hoher Nullscherviskositäten so hohe Konzentrationen erforderlich, dass verträgliche Osmolalitäten nicht eingehalten werden könnten.
Wu C. et a.l, Polymer Bulletin, Bd. 48, Nr. 1, 2002, Seiten 33-41, beschreiben ein Ethylcellulosederivat mir mesogenen Gruppen durch Umsetzung von Ethylcellulose mit 4-Methoxyphenyl-4-oxyessigsäurebenzoat.
Wu C. et al., Liquid Crystals, Bd. 30, Nr. 6, 2003, Seiten 733-737, beschreiben ein Ethylcellulosederivat, das Azobenzol-gebundene mesogene Gruppen enthält.
V.A.E. Shaik et al., Journal of Applied Polymer Science, Bd. 70, Nr. 1, 1998 , Seiten 195-201, beschreiben Cholesterin-gebundene Polysaccharide u.a. von Ethylcellulose.
V.A.E. Shaik et al., Journal of Applied Polymer Science, Bd. 72, Nr. 6, 1999 , Seiten 763-770, beschreiben Monocholesterylsuccinatderivate von Hydroxyethylcellulose.
V.A.E. Shaik et al., Journal of Applied Polymer Science, Bd. 100, Nr. 3, 2006, Seiten 1995-2001, beschreiben verschiedene mesogene Lithocholsäurederivate mit Hydroxyethylcellulose.
J.H. Kim et al., Polymer Korea, Bd. 28, Nr. 1, 2004, Seiten 92-102, beschreiben 6-Cholesteryloxycarbonylpentoxypropylcellulose.
Hu X. et al., Polymer, Bd. 45, Nr. 18, 2004, Seiten 6219-6225, beschreiben Azobenzol-funktionalisierte Hydroxypropylmethylcellulose.
Weiyan Wang et al, Polymer Bulletin, Bd. 59, Nr. 4, 2007, Seiten 537-544, beschreiben die Wirkung von a-Cyclodextrin auf die cis-trans-Photoisomerisierung von Azobenzolfunktionalisierter Hydroxypropylmethylcellulose.
Tianhui Hu et al., Cellulose, Bd. 17, Nr. 3, 2010, Seiten 547-558, beschreiben mit Methoxyazobenzol funktionalisierte Ethylcellulose.
Gallot B. et al., Liquid Crystals, Bd. 23, Nr. 1, 1997, Seiten 137-146, beschreiben die Struktur von Polylysin, das 20-100% Azobenzoleinheiten enthält.
DE 29 39 782 A1 beschreibt anisotrope Verbindungen mit 3 aromatischen Ringen und polarisierenden Seitengruppen.
DE 41 30 660 A1 beschreibt Flüssigkristallmaterial mit einer optischen Komponente, die drei cyclische Reste enthält.
Peijie Zheng et al., Macromolecular Rapid Communications, Bd. 25, Nr. 5, 2004, Seiten 678-682, beschreiben Azobenzol-funktionalisierte Hydroxypropylmethylcellulosederivate.

Vor dem Hintergrund dieses Standes der Technik haben sich die Erfinder die Aufgabe gestellt, Polysaccharidether bereitzustellen, die aus gut zugänglichen Rohstoffen hergestellt werden können, deren wässrige Lösungen unter Schereinwirkungen ein Fließverhalten ähnlich dem von Hyaluronsäuresalzlösungen aufweisen, bei gängigen Dampfsterilisationsverfahren keinen substantiellen Viskositätsabbau erleiden und ungekühlt transportiert und gelagert werden können, ohne einen Qualitätsverlust zu erleiden. Zugleich sollen insbesondere die anwendungsbezogenen Eigenschaften der Pseudoplastizität und Dispersivität bei hohen Nullscherviskositäten den klinischen Erfordernissen angepasst werden (Steve A. Arshinoff, Masoud Jafari; J. Cataract Refract. Surgery, Vol 31, 2005, 2167-2171 and B. Dick, O. Schwenn, N. Pfeiffer; Ophthalmologe, 1999, 193-211).

Gegenstand der Erfindung ist eine wie im Anspruch 1 definierte viskoelastische Zubereitung. Diese enthält wasserlösliche Polysaccharidether, insbesondere mit der Fähigkeit zur Aggregatbildung in wässriger Lösung, nämlich modifizierte Polysaccharidether mit einem gewichtsgemittelten Molekulargewicht von 40 000 bis 500 000 g/mol, einer Nullscherviskosität von mehr als 10 Pa. s und einer Pseudoplastizität von mehr als 20, erhältlich durch Umsetzen von Polysaccharidether(n), ausgewählt aus Hydroxypropylmethylcellulose (HPMC), Hydroxyethylmethylcellulose (HEMC), Methylcellulose, Celluloseethern mit Methyl- und/oder Ethyl- und/oder Propylgruppen und Gemischen davon, mit mindestens einem mesogenen Modifizierungsmittel.

Ein weiterer Gegenstand der Erfindung sind gattungsgemäße Produkte, deren Lösungen permanent farbig sind oder durch zusätzliche Substituenten Polyelektrolytcharakter erlangen.

Darüber hinaus sind Gegenstand der Erfindung viskoelastische Zubereitungen für die Injektion in den menschlichen Körper, die eines der modifizierten Polysacharide, oder eine Mischung dieser mit sich selbst oder anderen OVD, insbesondere auch Hyaluronsäure, sowie physiologisch akzeptable Salze und/oder ein Puffersystem sowie gewünschtenfalls weitere Wirk- und/oder Hilfsstoffe in wässriger Lösung enthalten.

Die überraschenden Fließeigenschaften, die die erfindungsgemäßen Produkte in wässriger Lösung zeigen, ließen sich durch keine wissenschaftliche Theorie prognostizieren. Insbesondere hatte der Fachmann nicht erwartet, dass Polysaccharidethern durch eine Modifizierung mit mesogenen Gruppen, insbesondere Cholesterylseitengruppen, viskoelastische Eigenschaften verliehen werden, die ansonsten nur von wässrigen Lösungen der Hyaluronsäure bekannt sind. Erklärungsversuche gehen davon aus, dass Polysaccharidether, etwa Hydroxypropylmethylcellulose, in wässriger Lösung sternförmige Fransenmicellen bilden. Es wird daher vermutet, dass die mesogenen Gruppen zu einer verstärkten Aggregation dieser Fransenmicellen führen. Überraschender Weise sind die dabei involvierten Wechselwirkungskräfte so geartet, dass schließlich das rheologische Verhalten von Hyaluronsäurelösungen erreicht wird, ohne dass deren Temperaturempfindlichkeit auftritt.

In einer sehr allgemeinen Ausführungsform betrifft die Erfindung viskoelastische Zubereitungen wasserlöslicher Polysaccharidether, die einem weiteren Modifizierungsschritt zur Einführung von mesogenen Gruppen unterworfen worden sind, wie im Anspruch 1 definiert. Wasserlöslich im Sinne der Erfindung sind Polysaccharidether, die zumindest bei Konzentrationen unter 10 Gew.-% und Temperaturen unter 40°C klare, durchsichtige Lösungen oder Gele bilden. Dabei können sich die Polysaccharidether von beliebigen Polysacchariden gemäß Anspruch 1 ableiten. Die Polysaccharide leiten sich von Glucan ab. Es sind dies Polysaccharide auf Basis von Glucose. Als Beispiele seien hier genannt Glucane mit α-1,4-glycosidischer Bindung, wie α-Amylase, und Glucane mit β-1,4-glycosidischer Bindung, wie Cellulose. Weitere Glucane für diesen Zweck sind Amylopektin, Callose oder Chitosan (teilweises deacetyliertes Chitin). Ein weiteres Polysaccharid ist Polygalactomannan, das üblicherweise aus Guarkernmehl gewonnen wird oder aus Johannisbrotkernmehl. Weitere Polysaccharide für diesen Zweck sind Dextran, Xanthan sowie Alginate.

Die erfindungsgemäßen Polysaccharidether sind Veretherungsprodukte der in Anspruch 1 genannten Polysaccharide. Als Veretherungsmittel werden hier die üblichen großtechnisch verwendeten Veretherungsmittel eingesetzt. Es sind dies Halogenalkane mit bevorzugt 1 bis 3, aber meist nicht mehr als 6 C-Atomen oder Epoxide, wie Ethylenoxid oder Propylenoxid, oder deren höhere Homologe (z.B. mit bis zu 7 C-Atomen), wie zum Beispiel die 1-Olefinoxide. Technisch gängig ist der Einsatz von Methylchlorid, Ethylchlorid einzeln oder zusammen mit einem oder beiden der genannten Epoxide. Großtechnisch hergestellte bekannte Produkte sind Methylcellulose, Ethylcellulose, Hydroxyethylcellulose, Hydroxypropylcellulose sowie die analogen Stärkederivate. Bekannt und gängig sind auch Mischformen, wie Hydroxyethylmethylcellulose, Hydroxypropylmethylcellulose bzw. die analogen Stärkeprodukte. Eine weitere wichtige Stoffklasse sind die Veretherungsprodukte von Cellulose, Stärke oder anderen Polysacchariden mit Chloressigsäure. Auf diesem Wege entstehen Produkte, wie Carboxymethylcellulose bzw. Carboxymethylstärke. Derartige funktionalisierte Polysaccharidether können weitere Substituenten, wie Methyl- oder Hydroxyethyl- oder Hydroxypropyl-Gruppen, tragen.

Für die technischen Eigenschaften der Polysaccharidether, die hier als Ausgangsstoffe der erfindungsgemäßen substituierten modifizierten Polysaccharidether beschrieben werden, ist der Substitutionsgrad von Bedeutung. Dieser gibt an, wie viele Substituenten im Mittel pro Monomer-Baustein vorhanden sind. Im Falle der Cellulose ist der Monomerbaustein die Anhydroglucoseeinheit. Diese verfügt über drei freie OH-Gruppen. Daraus ergibt sich, dass für Alkylsubstituenten oder für die Carboxymethylgruppe der theoretische, maximale Substitutionsgrad 3 beträgt. Dieser wird jedoch technisch nie erreicht. Für den praktischen Gebrauch liegen die Substitutionsgrade zwischen 0,1 und 2,5.

Im Falle der Modifizierung mit Ethylenoxid bzw. Propylenoxid entsteht aus einer freien OH-Gruppe des Makromoleküls eine neue freie OH-Gruppe. Daher kann bei Substitution mit Ethylenoxid bzw. Propylenoxid auch ein scheinbarer Substitutionsgrad > 3 eingestellt werden. Praktisch bedeutet dies, dass eine Anhydroglucoseeinheit mit mehr als 3 Molekülen Ethylenoxid bzw. Propylenoxid substituiert ist. Diese Moleküle lagern sich aber nicht unbedingt direkt an den Anhydroglucosering an, sondern können auch Oligo- bzw. Polyetherseitengruppen bilden, indem sie sich an die jeweils neu entstandene OH-Gruppe weiter anlagern.

Besonders bevorzugte Materialien im Sinne der Erfindung leiten sich von gemischten Polysaccharidethern ab, wie beispielsweise von Alkylhydroxyethylcellulosen bzw. Alkylhydroxypropylcellulosen. Besonders bevorzugte Materialien sind Hydroxypropylmethylcellulose und Hydroxyethylmethylcellulose.

Ideale Ausgangsmaterialien für die erfindungsgemäßen neuen Polysaccharidether sind Alkylhydroxyethylcellulosen bzw. Alkylhydroxypropylcellulosen, wie sie für pharmazeutische Zwecke von vielen Herstellern angeboten werden. Unter diesen hat unter praktischen Gesichtspunkten Hydroxypropylmethylcellulose die weiteste Verbreitung gefunden. Ein besonders bevorzugtes Ausgangsmaterial ist eine gereinigte Hydroxypropylmethylcellulose, wie sie als synthetisches viskoelastisches Material für ophthalmologische Anwendungen empfohlen wird und im US-Patent 5,422,376, Spalte 5, Zeile 20 bis Spalte 6, Zeile 64 beschrieben ist.

Für die Zwecke der hier vorliegenden Erfindung gilt allgemein, dass solche Polysaccharidether bevorzugt sind, die in heterogener Phase hergestellt worden sind. Dies gilt für die meisten technischen Produkte, die im Handel sind. Durch die Herstellung in heterogener Phase findet die Substitution der OH-Gruppen am Polysaccharidmolekül bevorzugt in den (im polymerchemischen Sinne) nicht kristallisierten Bereichen statt. Die Produkte erhalten dadurch eine Art Blockcopolymerstruktur, d.h. in den ursprünglich nicht kristallinen Bereichen sind mehr Substituenten als in den Bereichen, die als Kristallite während des Herstell-Prozesses nur schwer durch die Alkylierungsmittel erreicht worden sind. Ohne dass exaktes Wissen darüber vorliegt, wird angenommen, dass eine solche Struktur die Bildung der eingangs genannten Fransenmicellen fördert. Besonders bevorzugte erfindungsgemäße Produkte bilden in wässriger Lösung derartige Fransenmicellen.

Hervorragende Ausgangsmaterialien für die nachfolgend beschriebene Modifizierung sind Produkte mit einem Alkylsubstitutionsgrad zwischen 0,5 und 2,3, insbesondere zwischen 1,0 und 2,0 und/oder einem Hydroxyalkylsubstitutionsgrad zwischen 0,3 bis 1,5. Liegt sowohl eine Alkylsubstitution als auch eine Hydroxyalkylsubstitution vor, so sollte der Alkylsubstitutionsgrad 0,8 bis 2,2, vorzugsweise 1,0 bis 2,0, betragen und gleichzeitig der Hydroxyalkylsubstitutiongrad 0,05 bis 1,0, vorzugsweise 0,1 bis 0,3. Der Fachmann wird bei Celluloseethern eher mittlere bis höhere Substitutionsgrade, bei Stärkeethern eher niedrigere Substitutionsgrade wählen.

Das Molekulargewicht der modifizierten Polysaccharide
der erfindungsgemäßen Zubereitung kann der Fachmann in
weiten Grenzen wählen. Es richtet sich im Allgemeinen nach der natürlichen Quelle für das Polysaccharid. Da die Viskosität der Zubereitung ein entscheidender Parameter ist, ist es vorteilhaft, Produkte mit möglichst hohem Molekulargewicht zu wählen. Übliche mittlere Molekulargewichte für Celluloseether liegen zwischen 10000 und 500000 Dalton. Soweit diese in dieser Anmeldung beschrieben werden, handelt es sich um das Gewichtsmittel.

Erfindungsgemäß werden die Polysaccharidether durch Einführen einer mesogenen Gruppe modifiziert. Der Begriff der mesogenen Gruppe ist dem Fachmann bekannt. Darunter werden alle Gruppierungen oder Substanzen verstanden, die geeignet sind, in einem bestimmten Temperaturbereich Mesophasen zu bilden, d.h. Phasen mit einem höheren Ordnungszustand als in isotropen Flüssigkeiten, jedoch mit einem niedrigeren als in Kristallgittern. Solche Phasen zeigen die optische Anisotropie von Kristallen und die Beweglichkeit von durchweg isotropen Flüssigkeiten. Mesophasen werden auch als flüssig-kristalline Phasen oder anisotrope Flüssigkeiten bezeichnet.

Die mesogene Gruppe kann im Rahmen der Erfindung eine solche sein, die zur Bildung smektischer, nematischer oder cholesterischer flüssig-kristalliner Phasen geeignet ist. Dies können somit stäbchenförmige, scheibenförmige (diskotische) oder cholesterische mesogene Gruppen sein.

Dem auf dem Gebiet flüssig-kristalliner Verbindungen tätigen Chemiker bieten sich zahlreiche flüssig-kristalline (mesogene) Verbindungen an, die über funktionelle Gruppen verfügen und in einfacher Weise angebunden werden können.

Eine Vielzahl geeigneter aromatischer und aliphatischer mesogener Substanzen sind in der Europäischen Patentanmeldung EP1440974 als Rest M (mesogener Rest) in den Abschnitten 19 bis 28 beschrieben. Auf diese Publikation wird hiermit expressis verbis Bezug genommen.

Erfindungsgemäß werden die mesogenen Gruppen als Seitenkette der Polysaccharidmoleküle angebracht. Die mesogene Struktur kann direkt an die Kette gebunden sein, oder über eine kürzere oder längere Zwischengruppe. Als besonders bevorzugt im Sinne der Erfindung können solche mesogenen Seitengruppen gelten, die stark hydrophob sind, eine möglichst geringe Wasserlöslichkeit aufweisen und / oder aus mehr als 12, vorzugsweise mehr als 30, C-Atomen aufgebaut sind. So sind beispielsweise Seitengruppen aus den folgenden Ausgangsmolekülen geeignet:
Cholesteryl-Derivate, wie:
   Cholesterylchlorformiat, Cholesterylhemisuccinat, Cholesterylchlorid, Cholesterol, Cholestan-3-ol, sowie 5,6-Dibromcholestan-3-ol.
Zyklische Terpene, wie:
   α-Tocopherol, Podocarpinsäure, Abietinsäure, Dehydroabietylamin.
Steroide, wie:
   Hydrocortison, β-Sisosterol, 19-Hydroxy-4-androsten-3,17-dion, Mestranol, Stearylglycyrrhetinat

Nach einer bevorzugten Ausführungsform der Erfindung wird als mesogene Gruppe Cholesterol eingeführt. Cholesterol oder eines seiner Derivate verfügt über eine OH-Gruppe und kann über eine geeignete difunktionelle Zwischengruppe an das Polymer gebunden werden. Dabei kann der Fachmann den Rest direkt an die Polymerkette anhängen oder über eine längere, als Abstandshalter fungierende Zwischengruppe. Beispielsweise kann die direkte Anbindung über eine Kohlensäureestergruppe erfolgen. Dazu wird Cholesterol im Molverhältnis 1:1 mit Phosgen umgesetzt und das so hergestellte Chlorformyl-Derivat gewünschtenfalls in Gegenwart von basischen Katalysatoren oder unter Einsatz elektromagnetischer Strahlung, wie Mikrowellen, an das Polymer gebunden. In ähnlicher Weise kann eine Bindung aber auch über andere difunktionelle Gruppen erfolgen, etwa über reaktive Verbindungen von Dicarbonsäuren oder über α-, Ω-Dihalogenalkane, Diepoxide oder dergleichen.

Als weitere oder alternative mesogene Gruppen sind synthetische mesogene Gruppen geeignet. Dabei handelt es sich um anisotrope Moleküle, die meistens aus einem starren aromatischen System bestehen, an das sich flexible Endgruppen anschließen. Eine Vielzahl mesogener Gruppen, die auch für die hier vorliegende Erfindung geeignet sind, sowie Wege zu ihrer Anbindung an Polymere sind beispielsweise in der Europäischen Patentanmeldung EP1440974 beschrieben. Besonders geeignet sind hier vollsynthetische mesogene/flüssigkristalline Substituenten wie:
1-(4-*trans*-Hexylcyclohexyl)-4-[2-(4-isothiocyanatophenyl)ethyl]benzol, 1-[4-(*trans*-4-Heptylcyclohexyl)phenyl]-2-[*trans*-4-(4-isothiocyanatophenyl)cyclohexyl]ethan, 4-Hexyl-4'-[2-(4-isothiocyanatophenyl)ethyl]-1,1'-biphenyl, 4-Isothiocyanatophenyl-4-pentylbicyclo[2.2.2]octan-1-carboxylat, 4-[(S,S)-2,3-Epoxyhexyloxy]phenyl-4-(decyloxy)benzoat, 6-[4-(4-Cyanophenyl)phenoxy]hexylmethacrylat, 4'-Brommethyl-2-biphenylcarbonitril. Weitere geeignete Substanzen sind im Chemikalienhandel erhältlich.

Der Substitutionsgrad der erfindungsgemäßen wasserlöslichen Polysaccharidether beträgt in Bezug auf die mesogenen Gruppen 0,0001 bis 0,2, vorzugsweise 0,001 bis 0,1. Dies bedeutet, dass pro Anhydroglucoseeinheit im Mittel 0,0001 bis 0,2 bzw. vorzugsweise 0,001 bis 0,01 mesogene Gruppen vorhanden sind.

Nach einer weiteren bevorzugten Ausführungsform der Erfindung enthält der erfindungsgemäße Polysaccharidether weitere modifizierende Seitengruppen. Als solche weiteren modifizierenden Seitengruppen können Farbstoffgruppen eingesetzt werden. Dem organischen Chemiker steht eine große Vielzahl von Farbstoffen zur Verfügung, die direkt kovalent an die OH-Gruppen von Polysacchariden gebunden werden können. Die Auswahl eines Farbstoffes kann sich daher nach dem Farbton einerseits (Lichtabsorption) und nach medizinischen Gesichtspunkten richten. Bevorzugt sind daher Farbstoffe, die eine ausreichende Biokompatibilität besitzen und von denen beispielsweise kein allergenes Potenzial bekannt ist.

Die Modifizierung der erfindungsgemäßen Produkte kann aus zwei Gründen erforderlich sein. Zum einen werden die erfindungsgemäßen Produkte bevorzugt bei Operationstechniken am menschlichen Auge eingesetzt und müssen bei dieser Anwendung meist zum Ende der Operation möglichst quantitativ entfernt werden. Dazu ist es für den Operateur hilfreich, das eingesetzte Produkt an seiner Farbe zu erkennen. Gefärbte Produkte sind auch hilfreich, um den Schutz von sensiblen Bereichen zu kontrollieren und den Einsatz von Viskoelastika in schwer abzugrenzenden Bereichen zu steuern. Ein weiterer Grund ist, dass bei Augenoperationen einfallende oder eingekoppelte Strahlung durch farbiges Material kontrolliert (Vermeidung von lichtinduzierten Schädigungen der Netzhaut) bzw. dosiert werden kann, indem das Licht der eingesetzten Lichtquelle durch ein farbiges Material zumindest anteilig absorbiert wird.

Je nach gewünschter Anwendung ist die Menge des Farbstoffes zu steuern. In dem Falle, in dem der Farbstoff lediglich zur Markierung der eingesetzten viskoelastischen Flüssigkeiten dient, ist es bevorzugt, mit möglichst geringen Farbstoffmengen hoher Absorptionskraft zu arbeiten. Nach einer besonders bevorzugten Ausführungsform der Erfindung können hier fluoreszierende Farbstoffe eingesetzt werden, da die Fluoreszenzstrahlung besonders leicht zu detektieren ist. In der Praxis hat sich insbesondere gezeigt, dass Fluoresceinderivate geeignet sind, so z.B. Fluoresceinderivate mit Isothiocyanatgruppen, die im Handel erhältlich sind und an die Polymere als Carbamothioat angehängt werden können.

Im Falle der Verwendung von Farbstoffen zur Absorption schädlicher Lichtenergiemengen sind höhere Substitutionsgrade erforderlich, die der Fachmann direkt aus der erforderlichem Lichtabsorption ermitteln kann.

Der auf diesem Gebiet tätige Fachmann wird für diesen Verwendungszweck insbesondere Reaktivfarbstoffe verwenden, wie diese im Handel für die Anfärbung von Baumwolle und anderen Cellulosederivaten erhältlich sind.

Verwiesen sei in diesem Zusammenhang auch auf die Internationale Anmeldung WO86/02548, in der ein Farbstoff beschrieben wird, der sich an ein Polysaccharid, in diesem Falle Dextran, binden lässt. Ein Vorteil der vorliegenden Erfindung gegenüber diesem Stand der Technik liegt darin, dass erfindungsgemäß der Farbstoff direkt an das dem viskoelastischen Material zugrunde liegende Polymer gebunden ist und nicht an ein weiteres Polymer, welches sich unter bestimmten Bedingungen anders verhalten, entmischen oder an spezielle Zellsysteme anlagern kann, was hier nicht gewünscht ist.

Nach einer weiteren Ausführungsform der Erfindung können die erfindungsgemäßen Produkte auch Polyelektrolytcharakter besitzen. Dies kann z. B. durch Einführung von Carboxymethylgruppen geschehen.

Bei der Modifizierung der Polysaccharidether mit den mesogenen Substanzen und/oder mit den Farbstoffen hat es sich als besonders bevorzugt erwiesen, in homogener Lösung zu arbeiten. Dazu werden die Celluloseether in einem Lösungsmittel, wie Dimethylsulfoxid oder dergleichen, gelöst und mit 0,1 Mol-%, bezogen auf Celluloseether eines Amins, z. B. Triethanolamin, versetzt. Zu diesem Reaktionsmedium wird dann das Mesogen, z. B. in Gestalt von Cholesterylchlorformiat, in einem geeigneten Lösungsmittel oder der Farbstoff, gelöst in einer 0,1-molaren Natronlauge, zugegeben. Um für eine bessere Durchmischung des Reaktionsmediums zu sorgen, hat es sich als vorteilhaft herausgestellt, diese Reaktionsmischung zu rühren oder auch in einem Kneter zu bearbeiten.

Nach einer weiteren Ausführungsform der Erfindung werden zur Verbesserung der Eigenschaften der viskoelastischen Zubereitungen unlösliche Teile abgetrennt, wie dies in dem eingangs zitierten US-Patent 5,422,376 beschrieben ist.

Aus dem erfindungsgemäßen Polysaccharidether lassen sich in einfacher Weise viskoelastische Zubereitungen, wie sie für die Augenheilkunde gebraucht werden, herstellen. Dazu löst man sie in wässrigen Salzlösungen, die gegebenenfalls auch noch einen geeigneten Puffer enthalten. Die zu lösende Menge an Polysaccharidether richtet sich nach der gewünschten Viskosität. Sie kann 0,05 bis 5 Gew.-% betragen, bevorzugt sind 1 bis 3 Gew.-%. Besonders günstig haben sich Mengen von um 2 Gew.-% erwiesen. Vom Fachmann kann die erforderliche Konzentration leicht an die bekannten Anwendungsgebiete von Hyaluronsäurepräparaten angepasst werden. Die hier eingesetzten Salze dienen der Einstellung des osmotischen Drucks. Geeignete Salze sind hier Natriumchlorid, Kaliumchlorid, Calciumchlorid und Magnesiumchlorid oder andere physiologisch akzeptable Salze. Dabei liegt der Salzgehalt oftmals unter 1,5 Gew.-%. Erfindungsgemäß werden weiterhin Puffersysteme beigefügt, vorzugsweise auf Basis von Natriumacetat und/oder Natriumcitrat. Mit Salzsäure oder Natronlauge wird, falls erforderlich, auf einen pH-Wert zwischen 5,5 und 8,5 eingestellt, für ophthalmologische Präparate vorzugsweise zwischen 6,8 und 8,1. Die Gesamtpuffermenge bewegt sich dabei in der Regel um oder unter 1 Gew.-%. Die Osmolalität wird dadurch zwischen 200-400 milliosmol eingestellt.

Als Anhaltspunkt für eine geeignete viskoelastische Lösung können die Zubereitungen angesehen werden, die in den Kapiteln 26 bis 28 der Europäischen Patentschrift EP1263363B1 beschrieben sind.

Bei der Herstellung der viskoelastischen Lösung ist weiterhin darauf zu achten, dass durch starke Scherkräfte das Molekulargewicht nicht vorzeitig abgebaut wird. Es hat sich daher gezeigt, dass ein bevorzugtes Verfahren darin besteht, die vorgefertigten, festen Polysaccharidether nach der Modifizierung zu isolieren, gewünschtenfalls zu vermahlen und die feste Zubereitung dann gewünschtenfalls nach einem Reinigungsschritt zur Abtrennung nicht gewünschter oder störender niedermolekularer Beimengungen in einem Nicht-Lösemittel, wie etwa heißem Wasser, zu dispergieren, und danach durch Abkühlen der wässrigen Flotte die Dispersion in eine Lösung zu überführen, woraufhin sich ein Abpackungs- und Sterilisationsschritt anschließen kann.

Die erfindungsgemäßen viskoelastischen Zubereitungen sind für die Injektion in den menschlichen Körper geeignet. Ein Hauptanwendungszweck liegt auf dem Gebiet der Ophthalmologie. Dort können die Zubereitungen z.B. bei Kataraktoperationen eingesetzt werden. Neben diesem Haupteinsatzgebiet sind die erfindungsgemäßen viskoelastischen Zubereitungen aber auch geeignete Hilfsmittel in der kosmetischen Chirurgie. So können sie, ähnlich wie Hyaluronsäurelösungen, zum Unterspritzen von Falten bei kosmetischen Behandlungen eingesetzt werden. Eine weitere Einsatzmöglichkeit liegt auf dem Gebiet der Orthopädie. Dort können die Zubereitungen ebenfalls in Analogie zur Hyaluronsäure als Ergänzung der Synovialflüssigkeit, zur Verbesserung der viskösen Konsistenz, der Absorption von Stoßbelastungen und Verbesserung der Gleitfähigkeit von Knorpeln und damit der verbesserten Beweglichkeit von Gelenken eingesetzt werden und dazu direkt in die Gelenkkapsel gespritzt werden. Die erfindungsgemäßen Produkte zeigen bevorzugt eine Nullscherviskosität > 100 Pa.s, sie sind dampfsterilisierbar und ohne Kühlung lagerbar und transportierbar.

Bevorzugte Bereiche für die Nullscherviskosität betragen 10 bis 20 000 Pa.s, vorzugsweise 10 bis 10 000 Pa.s, bevorzugter 10 bis 5000 Pa.s, zudem bevorzugt 10 bis 1000 Pa.s. Weitere bevorzugte Bereiche von Nullscherviskositätswerten sind von 20 bis 1000 Pa.s, bevorzugter 50 bis 1000 Pa.s, zudem bevorzugt 100 bis 1000 Pa.s, weiterhin bevorzugt 500 bis 1000 Pa.s.

Bevorzugte Bereiche für die Pseudoplastizität der erfindungsgemäßen Produkte betragen 20 bis 10 000, vorzugsweise 20 bis 5000, bevorzugter 20 bis 2000, zudem bevorzugt 20 bis 1000. Weitere bevorzugte Bereiche von Pseudoplastizitätswerten sind von 50 bis 1000, bevorzugter 100 bis 1000, zudem bevorzugt 200 bis 1000, weiterhin bevorzugt 400 bis 1000.

Unter Berücksichtigung der Struktur des jeweiligen Cellulose-Derivates korreliert die Pseudoplastizität mit dem Molekulargewicht.

### Beispiel 1

Derivatisierung von HPMC mit Cholesterylchlorformiat
5,0 g (24,7 mmol) getrocknete HPMC und 554 mg (1,23 mmol) Cholesterylchlorformiat wurden in einer Stickstoffatmosphäre bei 75°C unter Nutzung eines KPG-Rührers in 50 ml trockenem DMSO gelöst. Anschließend wurden 345 µl (2,47 mmol) Triethylamin zugegeben und 16 Stunden bei 75°C gerührt. Das gelöste Polymer wurde mit 50 ml DMSO verdünnt und heiß in einem Gemisch aus Diethylether/Ethanol (4:1) ausgefällt. Der Feststoff wurde per Filtration abgetrennt, mit Aceton gewaschen und anschließend mit Aceton über 48 Stunden extrahiert. Man erhielt 5,1 g eines farblosen Feststoffs (92 % der Theorie).
¹H-NMR (200MHz, DMSO-d6, 100°C): δ= 4,54-4,26 (*breit,* C¹*H*, 291H), 3,9-2,6 *(breites Multiplett),* 1,13-1,00 (*breit,* -OCH₂CHOHC*H*₃, 225H), 0,91 (d, C²⁷*H*₃, 3H), 0,89-0,86 (m, C¹⁹*H₃*, C²⁶*H₃*, C²⁸*H₃*, 9H), 0,69 (s, C²⁰*H₃*, 3H) ppm.
Durch das Verhältnis der Integrale des C¹*H*-Signals des Glucosids und des C²⁰*H₃*-Signals des Cholesterylsubstituenten ergab sich ein Substitutionsgrad von DS=1,15·10⁻³ (≃ 0,74 Gew.-%). Die Bestimmung des Molekulargewichts lieferte eine bimodale Verteilung mit den beiden Maxima bei M₁ = 103 000 g/mol und bei M₂ = 1 690 000 g/mol. Diese Molmassen repräsentieren nicht das Molekulargewicht monodispers gelöster Ketten. Dem Fachmann ist bekannt, dass insbesondere hydrophob modifizierte Celluloseether Aggregate bilden, die unter den für die GPC-Messungen angewendeten Messbedingungen vollständig oder teilweise erhalten bleiben. Die Erhöhung des Molekulargewichts kann entsprechend auf eine verstärkte Aggregation zurückgeführt werden. Diese Beschränkung gilt für alle experimentell ermittelten Molmassen.

Die 2 gew.-%-ige Lösung in Wasser hatte eine Trübungstemperatur von 63,0°C, eine Nullviskosität von 1300 Pa.s und eine Pseudoplastizität von 229. Die Nullviskosität war gegenüber der unmodifizierten HPMC um den Faktor 60 gesteigert, die Pseudoplastizität um den Faktor 10.
Entsprechend eines für pharmazeutische Anwendungen standardmäßig verwendeten Herstellungsverfahrens wird eine 2 gew.-%-ige Lösung der Cholesterylmodifizierten HPMC in physiologischem Puffer in 2,25 ml Spritzen abgefüllt und anschließend in einem Dampfautoklaven bei 121°C für 20 min sterilisiert. Die Nullviskosität dieser Lösung nahm dabei auf 750 Pa.s ab, die Pseudoplastizität auf 431 zu. Die Nullviskosität einer Vergleichsprobe von Hyaluronsäure (1,5 Gew.-% in physiologischem Puffer, Nullviskosität η₀=640 Pa.s) nahm auf 8,3 % ab.

Alle im Folgenden genannten Beispiele werden entsprechend der oben genannten Vorschrift synthetisiert.

### Beispiel 2

Derivatisierung von HEMC mit Cholesterylchlorformiat
¹H-NMR (200MHz, DMSO-d6, 100°C): δ = 4,54-4,26 (*breit,* C¹*H*, 281H), 3,9-2,6 *(breites Multiplett),* 0,91 (d, C²⁷*H₃*, 3H), 0,89-0,86 (m, C¹⁹*H₃*, C²⁶*H₃,* C²⁸*H₃*, 9H), 0,69 (s, C²⁰*H₃*, 3H) ppm.
Durch das Verhältnis der Integrale des C¹*H*-Signals des Glucosids und des C²⁰*H₃*-Signals des Cholesterylsubstituenten ergab sich ein Substitutionsgrad von DS=1,19·10⁻³ (≃0,73 Gew.-%). Die Bestimmung des Molekulargewichts lieferte eine trimodale Verteilung mit den Maxima bei M₁ = 113 000 g/mol, M₂ = 438 000 g/mol und M₃= 1 435 000 g/mol. Die 2 gew.-%-ige Lösung in Wasser hatte eine Trübungstemperatur von 60,3°C, eine Nullviskosität von 184 Pa.s und eine Pseudoplastizität von 356.

### Beispiel 3

Derivatisierung von HEMC mit 4-(4-Decyloxybenzoyloxy)benzoesäurechlorid
¹H-NMR (200MHz, DMSO-d6, 100°C): δ = 5,63-5,33 (*breit, OH*), 4,56-4,26 *(breit,* C¹*H*), 4,0-2,6 (*breites Multiplett*) ppm.
UV-Vis (H₂O): λₘₐₓ(A) = 264(0,573) nm.
Via UV-Spektroskopie ließ sich der Substitutionsgrad zu DS=1,35·10⁻³ (≃ 0,76 Gew.-%) bestimmen (ε₂₆₄ₙₘ= 14325 cm²/mmol). Die Bestimmung des Molekulargewichts lieferte eine bimodale Verteilung mit den Maxima bei M₁ = 242 000 g/mol und M₂ = 445 000 g/mol. Die 2 gew.-%-ige Lösung in Wasser hatte eine Trübungstemperatur von 61,7°C, eine Nullviskosität von 28,2 Pa.s und eine Pseudoplastizität von 22.

### Vergleichsbeispiel 1

Derivatisierung von HPMC mit Octylchlorformiat
DS=2,56·10⁻³ (≃ 0,63 Gew.-%). Die Bestimmung des Molekulargewichts liefert eine bimodale Verteilung mit den beiden Maxima bei M₁ = 118 000 g/mol und bei M₂ = 403 000 g/mol. Die 2 gew.-%-ige Lösung in Wasser hat eine Trübungstemperatur von 68,3°C, eine Nullviskosität von 2,9 Pa.s und eine Pseudoplastizität von nur 2,8. ¹H-NMR (200MHz, DMSO-d6, 100°C): δ = 6,25-5,60 (*breit,* O*H*) ,4,54-4,26 *(breit,* C¹*H*, 130H), 3,9-2,7 (*breites Multiplett*), 1,38-1,23 (m, C³⁻⁷*H*₂, 10H), 1,13-1,00 (*breit,-*OCH₂CHOHC*H₃*, 92H) ppm.
Durch das Verhältnis der Integrale des C¹*H*-Signals des Glucosids und des Signals der Methylengruppen C³⁻⁷*H₂* des Octylsubstituenten ergibt sich ein Substitutionsgrad von DS=2,56·10⁻³ (≃ 0,63 Gew.-%). Die Bestimmung des Molekulargewichts liefert eine bimodale Verteilung mit den beiden Maxima bei M₁ = 118 000 g/mol und bei M₂ = 403 000 g/mol. Die 2 gew.-%-ige Lösung in Wasser hat eine Trübungstemperatur von 68,3°C, eine Nullviskosität von 2,9 Pa.s und eine Pseudoplastizität von 2,8. Die Nullviskosität ist gegenüber der unmodifizierten HPMC um den Faktor 10 gesunken.

### Vergleichsbeispiel 2:

Derivatisierung von HPMC mit Octadecylisocyanat (Hydrophobe Verbindung, aber kein Mesogen)
¹H-NMR (200MHz, DMSO-d6, 100°C): δ = 5,95-5,30 (*breit, OH*), 4,60-4,30 *(breit,* C¹*H*, 34,1H), 3,9-2,7 *(breites Multiplett),* 1,51-1,43 (*breit,* C²*H*₂, 2H),1,40-1,25 (m, C³⁻¹⁷*H₂*, 30H), 1,16-1,00 (*breit,* -OCH₂CHOHC*H*₃, 23,1H), 0,94-0,86 (*breit,* C¹⁸*H₃*, 3H) ppm.
Durch das Verhältnis der Integrale des C¹*H*-Signals des Glucosids und des Signals der Methylengruppen C³⁻¹⁷*H₂* des Octadecylsubstituenten ergibt sich ein Substitutionsgrad von DS=9,78·10⁻³ (≃ 4,37 Gew.-%). Dieser modifizierte Polysaccharidether ist vollständig wasserunlöslich.

### Analytische Methoden:

### Rheometrie

Rheologische Messungen werden an einem Haake Marsll-Rheometer der Fa. Fisher scientific im controlled stress Modus mit einem Platte-Platte Aufbau durchgeführt (Sensor-Platte: PP35Ti; Messplatte: MP35). Vermessen werden 2 gew.-%-ige wässrige Lösungen nach der Europäischen Norm EN ISO 15798:2001 für ophthalmologische Gleitmittel: Entsprechend wird bei einer Temperatur von 25 ±0,1 °C gearbeitet. Dynamische Viskositäten werden im Bereich von *γ̇* = 0,002 bis 1000s⁻¹ aufgenommen. Nullviskositäten werden nach dem Newton-Modell im Bereich von *γ̇* = 0,002 bis 0,1 s⁻¹ bestimmt. Die Viskosität bei *γ̇* = 100 s⁻¹ wurde über die Extrapolation des power-law Bereichs von 10 ≤*γ̇*≤ 1000 s⁻¹ nach dem Modell von Ostwald de Waele berechnet. Die Pseudoplastizität ergibt sich durch das Verhältnis der Nullviskosität zur Viskosität bei *γ̇* = 100 s⁻¹.

### Trübungsphotometrie

Es werden 2 gew.-%-ige Lösungen der gereinigten Substanz in dest. Wasser in einer Standard-Quarzglasküvette vermessen. Messgerät ist ein Tepper-Trübungsphotometer mit LED- Lichtquelle (λ = 592 nm).
UV - Vis-Messungen
Es wird an einem Nicolet UV 540-Spektrometer gemessen. Datenpunkte werden mit einer Auflösung von 1 nm im Bereich von 200-500nm aufgenommen. HPMC-Proben werden als 0,2 gew.-%-ige wässrige Lösungen vermessen.
Molmassenbestimmung via Wasser-Gelpermeationschromatographie
Es werden 100 µl einer 0,1 gew.-%igen Lösung des betreffenden Polysaccharids in Wasser über eine HEMABio Trennsäule mit entsprechender Vorsäule getrennt. Messung per statische Lichtstreuung (miniDawn Treos, Fa. Wyatt) und des Brechungsindexinkrements (OptilabRex, Fa. Wyatt) ermöglichen die Bestimmung absoluter Molmassen.
¹H-NMR
Die Messungen erfolgen an einem FT-NMR-Spektrometer vom Typ Bruker Ultraflex DRX200 in DMSO-d6 bei 100°C. Zuvor werden die Celluloseketten durch Zugabe einer 10 Vol-% Trifluoressigsäurelösung in Wasser und Erhitzen auf 50°C für 3 Stunden abgebaut.

### Materialien:

Die Hydroxypropylmethylcellulose (HPMC) wird unter dem Handelsnamen Methocel®K15M Premium Hydroxypropylmethylcellulose von Colorcon bezogen. Der Massenanteil beträgt im Fall der Hydroxypropylierung 8,9 Gew.-% und für die Methylierung 22,8 Gew.-%. Die Bestimmung des Molekulargewichts liefert eine bimodale Verteilung mit den beiden Maxima bei M₁ = 134 000 g/mol und bei M₂ = 282 000 g/mol. Eine 2 gew.-%-ige Lösung in Wasser liefert eine Nullviskosität von 25 Pa.s und eine Pseudoplastizität von 17,3. Der Trübungspunkt dieser Lösung beträgt 70,8°C.
2-Hydroxyethylmethylcellulose (HEMC) wird von Aldrich bezogen.
Für HEMC beträgt der Massenanteil im Fall der Hydroxyethylgruppen 8,0 Gew.-% und für die Methylierung 26,0 Gew.-%. Die Bestimmung des Molekulargewichts liefert eine bimodale Verteilung mit den beiden Maxima bei M₁ = 156 000 g/mol und bei M₂ = 464 000 g/mol. Eine 2 gew.-%-ige Lösung in Wasser liefert eine Nullviskosität von 25 Pa.s und eine Pseudoplastizität von 12,8. Der Trübungspunkt dieser Lösung beträgt 63,9°C.
4-(4-Decyloxybenzoyloxy)benzoesäurechlorid wird entsprechend der bekannten Literatur synthetisiert. (Barbera, L. Puig, P. Romero, J. L. Serrano, T. Sierra, Journal of the American Chemical Society, Bd. 128, 2006, 4487-4492 and A. K. Singh, S. Kumari, T. N. G. Row, J. Prakash, K. R. Kumar, B. Sridhar, T. R. Rao, Polyhedron, Bd. 27, 2008, 3710-3716)
Cholesterylchlorformiat (puriss.; ≥99 %; Fluka), Octadecylisocyanat (tech.; Aldrich) und Octylchlorformiat (97 %; Aldrich) werden ohne weitere Reinigung eingesetzt. Alle Lösungsmittel haben technische Reinheit. DMSO wurde unter Argonatmosphäre über Molekularsieb 4 Å getrocknet.

### Molekulargewichtsbestimmung

Das Molekulargewicht wurde wie nachstehend ermittelt, sofern das Molekulargewicht nicht bereits aufgrund von Herstellerangaben bekannt ist. Die Bestimmung der Molmassenverteilung erfolgte über ein GPC-MALS-System, das aus den folgenden Komponenten besteht:

| | |
|---|---|
| Pumpe | Agilent 1200 Serie, bestehend aus Degasser, Pumpe und Autosampler; |
| Säule | Vorsäule HEMA Bio 300 + HEMA Bio 300, MZ-Analysentechnik |
| MALS-Detektor | miniDawn Treos; Wyatt; 3 Winkel |
| RI-Detektor | Optilab-Rex; Wyatt; |
| Laufmittel | Millipore-Wasser mit Zusatz von 100 mM NaNO₃, 500 ppm NaN₃; Das hierfür benötigte Brechungs-Indexinkrement (dn/dc) wurde mit dem Wert 0,15 der Datenbank von Wyatt.eu entnommen. |

Die Celluloseether wurden mit einer Massenkonzentration von 0,1 % im Laufmittel gelöst und es wurden 100 µl dieser Lösung in die Säule injiziert. Die Auswertung der Messsignale erfolgte über die Astra5-Software der Fa. Wyatt. Die Auswertung des Lichtstreusignals basiert auf dem Zimm-Modell.

## Patentansprüche

1. Viskoelastische Zubereitung, enthaltend 0,05 bis 5 Gew.-%, vorzugsweise 1 bis 3 Gew.-%, eines modifizierten Polysaccharidethers in wässriger Lösung, wobei der modifizierte Polysaccharidether ein gewichtsgemitteltes Molekulargewicht von 40 000 bis 500 000 g/mol aufweist, und erhältlich ist durch Umsetzen von Polysaccharidether(n), ausgewählt aus Hydroxypropylmethylcellulose (HPMC), Hydroxyethylmethylcellulose (HEMC), Methylcellulose, Celluloseethern mit Methyl- und/oder Ethyl- und/oder Propylgruppen und Gemischen davon, mit mindestens einem Modifizierungsmittel,
**dadurch gekennzeichnet,**
**dass** die viskoelastische Zubereitung eine Nullscherviskosität von mehr als 10 Pa.s, gemessen bei 25±0,1 °C und eine Pseudoplastizität, die sich durch das Verhältnis der Nullscherviskosität, gemessen bei 25±0,1 °C, zur Viskosität, gemessen bei 25±0,1 °C, und bei einer Schergeschwindigkeit von 100 s⁻¹ ergibt, von mehr als 20 aufweist, und dass das Modifizierungsmittel ein mesogenes Modifizierungsmittel ist.

2. Viskoelastische Zubereitung nach Anspruch 1 zur Injektion in den menschlichen Körper, insbesondere zur Verwendung bei operativen Eingriffen am menschlichen Auge, bei Verfahren der kosmetischen Chirurgie und/oder bei orthopädischchirurgischen Verfahren zur Ergänzung der Sinovialflüssigkeit und Verbesserung von Stoßabsorptionseigenschaften und Erhöhung der Gleitfähigkeit von Knorpeln und der Beweglichkeit von Gelenken.

3. Viskoelastische thermoresistente dampfsterilisierbare und ohne Kühlung lagerbare und transportierbare Zubereitungen nach Anspruch 1 oder 2, **dadurch gekennzeichnet, dass** die Nullscherviskositäten > 100 Pa.s, gemessen bei 25±0,1 °C, betragen.

4. Verfahren zur Herstellung einer viskoelastischen Zubereitung nach einem der Ansprüche 1 bis 3, wobei der modifizierte Polysaccharidether ein gewichtsgemitteltes Molekulargewicht von 40 000 bis 500 000 g/mol aufweist, und erhältlich ist durch Umsetzen von Polysaccharidether(n), ausgewählt aus Hydroxypropylmethylcellulose (HPMC), Hydroxyethylmethylcellulose (HEMC), Methylcellulose, Celluloseethern mit Methyl- und/oder Ethyl- und/oder Propylgruppen und Gemischen davon, mit mindestens einem Modifizierungsmittel,
**dadurch gekennzeichnet,**
**dass** man einen modifizierten Polysaccharidether in einem aprotischen Lösungsmittel löst oder dispergiert, mit einem oder mehreren Modifizierungsmitteln reagieren lässt, das so erhaltene modifizierte Polysaccharid isoliert, gewünschtenfalls niedermolekulare Anteile abtrennt, in Wasser löst, abpackt und sterilisiert.
**dass** die viskoelastische Zubereitung eine Nullscherviskosität von mehr als 10 Pa.s, gemessen bei 25±0,1 °C und eine Pseudoplastizität, die sich durch das Verhältnis der Nullscherviskosität, gemessen bei 25±0,1 °C, zur Viskosität, gemessen bei 25±0,1 °C, und bei einer Schergeschwindigkeit von 100 s⁻¹ ergibt, von mehr als 20 aufweist, und
**dass** das Modifizierungsmittel ein mesogenes Modifizierungsmittel ist.

5. Verfahren nach Anspruch 4, **dadurch gekennzeichnet, dass** man zur Herstellung wässriger Lösungen das Polysaccharid oberhalb der Lösungstemperatur in heißem Wasser dispergiert und die Dispersion durch Abkühlen in eine Lösung überführt.

6. Verwendung modifizierter Polysaccharidether mit einem gewichtsgemittelten Molekulargewicht von 40 000 bis 500 000 g/mol, einer Nullscherviskosität von mehr als 10 Pa.s, gemessen bei 25±0,1 °C, und einer Pseudoplastizität, die sich durch das Verhältnis der Nullscherviskosität, gemessen bei 25±0,1 °C, zur Viskosität, gemessen bei 25±0,1 °C, und bei einer Schergeschwindigkeit von 100 s⁻¹ ergibt, von mehr als 20, erhältlich durch Umsetzen von Polysaccharidether(n), ausgewählt aus Hydroxypropylmethyl-cellulose (HPMC), Hydroxyethylmethylcellulose (HEMC), Methylcellulose, Celluloseethern mit Methyl- und/oder Ethyl- und/oder Propylgruppen und Gemischen davon, mit mindestens einem Modifizierungsmittel, zur Herstellung einer viskoelastischen Zubereitung nach einem der Ansprüche 1 bis 3,
**dadurch gekennzeichnet,**
**dass** das Modifizierungsmittel ein mesogenes Modifizierungsmittel ist.

7. Verwendung modifizierter Polysaccharidether nach Anspruch 6, **dadurch gekennzeichnet, dass** der Substitutionsgrad von Hydroxypropylmethylcellulose (HPMC), definiert als Anzahl der Substituenten pro Anhydroglucoseeinheit für die Methylsubstituenten zwischen 0,8 bis 2,2, vorzugsweise 1,0 bis 2,0, und für die Hydroxypropylsubstituenten zwischen 0,05 und 1, vorzugsweise 0,1 und 0,3, liegt.

8. Verwendung modifizierter Polysaccharidether nach Anspruch 6, **dadurch gekennzeichnet, dass** der Substitutionsgrad von Hydroxyethylmethylcellulose (HEMC), definiert als Anzahl der Substituenten pro Anhydroglucoseeinheit für die Methylsubstituenten zwischen 0,8 bis 2,2, vorzugsweise 1,0 bis 2,0, und für die Hydroxyethylsubstituenten zwischen 0,05 und 1, vorzugsweise 0,1 und 0,3, liegt.

9. Verwendung modifizierter Polysaccharidether nach einem der Ansprüche 6 bis 8, **dadurch gekennzeichnet, dass** als mesogene Gruppen Cholesterylreste und/oder vollsynthetische mesogene Systeme, Steranderivate oder Terpenderivate vorliegen.

10. Verwendung modifizierter Polysaccharidether nach einem der Ansprüche 6 bis 9, **dadurch gekennzeichnet, dass** die mesogenen Systeme unmittelbar oder über eine Zwischengruppe an die Polymerkette gebunden sind.

11. Verwendung modifizierter Polysaccharidether nach einem der Ansprüche 6 bis 10, **dadurch gekennzeichnet, dass** die Anzahl der mesogenen Gruppen 0,0001 bis 0,2, vorzugsweise 0,001 bis 0,01 pro Anhydroglucoseeinheit beträgt.

12. Verwendung modifizierter Polysaccharidether nach einem der Ansprüche 6 bis 11, **dadurch gekennzeichnet, dass** es sich bei der mesogenen Gruppe um einen als Kohlensäureester gebundenen, gegebenenfalls derivatisierten Cholesterylrest handelt.

13. Verwendung modifizierter Polysaccharidether nach einem der Ansprüche 6 bis 12, **dadurch gekennzeichnet, dass** sie als weiteren Rest das Reaktionsprodukt eines gefärbten Modifizierungsmittels enthalten.

14. Verwendung modifizierter Polysaccharidether nach Anspruch 13, **dadurch gekennzeichnet, dass** sie als weiteres Modifizierungsmittel einen Reaktivfarbstoff oder ein Reaktionsprodukt eines reaktiven Fluoreszeinderivats kovalent gebunden enthalten.

15. Verwendung modifizierter Polysaccharidether nach einem der Ansprüche 6 bis 15 , **dadurch gekennzeichnet, dass** sie durch einen weiteren Rest Polyelektrolytcharakter besitzen.

## Claims

1. Viscoelastic preparation containing 0.05 to 5 wt.%, preferably 1 to 3 wt.%, of a modified polysaccharide ether in aqueous solution, wherein the modified polysaccharide ether has a weight-averaged molecular weight of 40,000 to 500,000 g/mol, and can be obtained by reacting polysaccharide ether(s), selected from hydroxypropyl methylcellulose (HPMC), hydroxyethyl methylcellulose (HEMC), methylcellulose, cellulose ethers with methyl groups and/or ethyl groups and/or propyl groups and mixtures thereof, with at least one modifier, **characterised in that** the viscoelastic preparation has a zero shear viscosity of more than 10 Pa.s, measured at 25±0.1 °C and a pseudoplasticity of more than 20 which is produced by the ratio of zero shear viscosity, measured at 25±0.1 °C, to the viscosity, measured at 25±0.1 °C, and at a shear rate of 100 s⁻¹, and **in that** the modifier is a mesogenic modifier.

2. Viscoelastic preparation according to claim 1 for injection into the human body, in particular for use in operative procedures on the human eye, in methods of cosmetic surgery and/or in methods of orthopaedic surgery for supplementing the synovial fluid and improving shock absorption properties and increasing the lubrication of cartilage and the flexibility of joints.

3. Viscoelastic heat-resistant, steam-sterilisable, transportable preparations which can be stored without cooling according to claim 1 or 2, **characterised in that** the zero shear viscosities are > 100 Pa.s, measured at 25±0.1 °C.

4. Method for producing a viscoelastic preparation according to one of claims 1 to 3, wherein the modified polysaccharide ether has a weight-averaged molecular weight of 40,000 to 500,000 g/mol, and can be obtained by reacting polysaccharide ether(s), selected from hydroxypropyl methylcellulose (HPMC), hydroxyethyl methylcellulose (HEMC), methylcellulose, cellulose ethers with methyl groups and/or ethyl groups and/or propyl groups and mixtures thereof, with at least one modifier, **characterised in that** a modified polysaccharide ether is dissolved or dispersed in an aprotic solvent, is allowed to react with one or more modifiers, the modified polysaccharide thus obtained is isolated, if required low-molecular portions are separated off, dissolved in water, packed and sterilised, **in that** the viscoelastic preparation has a zero shear viscosity of more than 10 Pa.s, measured at 25±0.1 °C and a pseudoplasticity of more than 20 which is produced by the ratio of zero shear viscosity, measured at 25±0.1 °C, to the viscosity, measured at 25±0.1 °C, and at a shear rate of 100 s⁻¹, and **in that** the modifier is a mesogenic modifier.

5. Method according to claim 4, **characterised in that** to produce aqueous solutions, the polysaccharide is dispersed in hot water above the solution temperature and the dispersion is transformed into a solution by cooling.

6. Use of modified polysaccharide ethers having a weight-averaged molecular weight of 40,000 to 500,000 g/mol, a zero shear viscosity of more than 10 Pa.s, measured at 25±0.1 °C, and a pseudoplasticity of more than 20 which is produced by the ratio of zero shear viscosity, measured at 25±0.1 °C, to the viscosity, measured at 25±0.1 °C, and at a shear rate of 100 s⁻¹, obtainable by reacting polysaccharide ether(s), selected from hydroxypropyl methylcellulose (HPMC), hydroxyethyl methylcellulose (HEMC), methylcellulose, cellulose ethers with methyl groups and/or ethyl groups and/or propyl groups and mixtures thereof, with at least one modifier to produce a viscoelastic preparation according to one of claims 1 to 3, **characterised in that** the modifier is a mesogenic modifier.

7. Use of modified polysaccharide ethers according to claim 6, **characterised in that** the degree of substitution of hydroxypropyl methylcellulose (HPMC), defined as the number of substituents per anhydroglucose unit, for the methyl substituents lies between 0.8 to 2.2, preferably 1.0 to 2.0, and for the hydroxypropyl substituents between 0.05 and 1, preferably 0.1 and 0.3.

8. Use of modified polysaccharide ethers according to claim 6, **characterised in that** the degree of substitution of hydroxyethyl methylcellulose (HEMC), defined as the number of substituents per anhydroglucose unit, for the methyl substituents lies between 0.8 to 2.2, preferably 1.0 to 2.0, and for the hydroxyethyl substituents between 0.05 and 1, preferably 0.1 and 0.3.

9. Use of modified polysaccharide ethers according to one of claims 6 to 8, **characterised in that** cholesterol radicals and/or fully synthetic mesogenic systems, androstane derivatives or terpene derivatives are present as mesogenic groups.

10. Use of modified polysaccharide ethers according to one of claims 6 to 9, **characterised in that** the mesogenic systems are bound to the polymer chain directly or via an intermediate group.

11. Use of modified polysaccharide ethers according to one of claims 6 to 10, **characterised in that** the number of mesogenic groups is 0.0001 to 0.2, preferably 0.001 to 0.01 per anhydroglucose unit.

12. Use of modified polysaccharide ethers according to one of claims 6 to 11, **characterised in that** the mesogenic group is an optionally derivatised cholesterol radical bound as a carbonate.

13. Use of modified polysaccharide ethers according to one of claims 6 to 12, **characterised in that** they contain as a further radical the reaction product of a dyed modifier.

14. Use of modified polysaccharide ethers according to claim 13, **characterised in that** they contain as a further modifier, a reactive dyestuff or a reaction product of a reactive fluorescein derivative covalently bound.

15. Use of modified polysaccharide ethers according to one of claims 6 to 15, **characterised in that** due to a further radical they have polyelectrolyte character.

## Revendications

1. Préparation viscoélastique, contenant 0,05 à 5 % en poids, de préférence 1 à 3 % en poids, d'un éther de polysaccharide modifié dans une solution aqueuse, dans laquelle l'éther de polysaccharide modifié présente un poids moléculaire pondéré de 40 000 à 500 000 g/mol et peut être obtenu par la mise en réaction d'éther(s) de polysaccharide, choisi(s) parmi l'hydroxypropylméthylcellulose (HPMC), l'hydroxyéthylméthylcellulose (HEMC), la méthylcellulose, les éthers de cellulose avec des groupes méthyle et/ou éthyle et/ou propyle et des mélanges de ceux-ci, avec au moins un agent de modification,
**caractérisée en ce**
**que** la préparation viscoélastique présente une viscosité à cisaillement nul supérieure à 10 Pa.s, mesurée à 25 ± 0,1 °C et une pseudoplasticité, qui résulte du rapport entre la viscosité à cisaillement nul, mesurée à 25 ± 0,1 °C, et la viscosité, mesurée à 25 ± 0,1 °C, et à une vitesse de cisaillement de 100 s⁻¹, de plus de 20, et que l'agent de modification est un agent de modification mésogène.

2. Préparation viscoélastique selon la revendication 1 destinée à être injectée dans le corps humain, en particulier destinée à être utilisée lors d'interventions chirurgicales sur l'œil humain, lors de procédures de la chirurgie cosmétique et/ou lors de procédures chirurgicales orthopédiques pour compléter le liquide synovial et améliorer des propriétés d'absorption de chocs et élever la capacité de glissement de cartilages et la mobilité d'articulations.

3. Préparations viscoélastiques thermorésistantes stérilisables à la vapeur et pouvant être stockées et transportées sans refroidissement selon la revendication 1 ou 2, **caractérisées en ce que** les viscosités à cisaillement nul sont > 100 Pa.s, mesurées à 25 ± 0,1 °C.

4. Procédé de fabrication d'une préparation viscoélastique selon l'une quelconque des revendications 1 à 3, dans lequel l'éther de polysaccharide modifié présente un poids moléculaire moyen de 40 000 à 500 000 g/mol et peut être obtenu par la mise en réaction d'éther(s) de polysaccharide, choisi (s) parmi l'hydroxypropylméthylcellulose (HPMC), l'hydroxyéthylméthylcellulos (HEMC), la méthylcellulose, les éthers de cellulose avec des groupes méthyle et/ou éthyle et/ou propyle et des mélanges de ceux-ci, avec au moins un agent de modification,
**caractérisé en ce**
**qu'**on dissout ou disperse un éther de polysaccharide modifié dans un solvant aprotique, on le laisse réagir avec un ou plusieurs agents de modification, on isole le polysaccharide modifié ainsi obtenu et on sépare si souhaité des fractions à faible poids moléculaire, on le dissout dans de l'eau, on le conditionne et on le stérilise,
**que** la préparation viscoélastique présente une viscosité à cisaillement nul supérieure à 10 Pa.s, mesurée à 25 ± 0,1 °C, et une pseudoplasticité, qui résulte du rapport entre la viscosité à cisaillement nul, mesurée à 25 ± 0,1 °C, et la viscosité, mesurée à 25 ± 0,1 °C, et à une vitesse de cisaillement de 100 s⁻¹, de plus de 20, et
**que** l'agent de modification est un agent de modification mésogène.

5. Procédé selon la revendication 4, **caractérisé en ce qu'**on disperse pour fabriquer des solutions aqueuses le polysaccharide à une température supérieure à la température de dissolution dans de l'eau très chaude et on transfère la dispersion par refroidissement dans une solution.

6. Utilisation d'éthers de polysaccharide modifiés avec un poids moléculaire moyen de 40 000 à 500 000 g/mol, une viscosité à cisaillement nul supérieure à 10 Pa.s, mesurée à 25 ± 0,1 °C, et une pseudoplasticité, qui résulte du rapport entre la viscosité à cisaillement nul, mesurée à 25 ± 0,1 °C, et la viscosité, mesurée à 25 ± 0,1 °C, et à une vitesse de cisaillement de 100 s⁻¹, de plus de 20, pouvant être obtenus par la mise en réaction d'éther(s) de polysaccharide, choisi(s) parmi l'hydroxypropylméthylcellulose (HPMC), l'hydroxyéthylméthylcellulos (HEMC), la méthylcellulose, les éthers de cellulose avec des groupes méthyle et/ou éthyle et/ou propyle et des mélanges de ceux-ci, avec au moins un agent de modification, pour fabriquer une préparation viscoélastique selon l'une quelconque des revendications 1 à 3,
**caractérisée en ce**
**que** l'agent de modification est un agent de modification mésogène.

7. Utilisation d'éthers de polysaccharide modifiés selon la revendication 6, **caractérisée en ce que** le degré de substitution de l'hydroxypropylméthylcellulose (HPMC), défini en tant que nombre des substituants par unité d'anhydroglucose pour les substituants de méthyle, se situe entre 0,8 à 2,2, de préférence 1,0 à 2,0, et pour les substituants d'hydroxypropyle entre 0,05 et 1, de préférence 0,1 et 0,3.

8. Utilisation d'éthers de polysaccharide modifiés selon la revendication 6, **caractérisée en ce que** le degré de substitution de l'hydroxyéthylméthylcellulose (HEMC), défini en tant que nombre des substituants par unité d'anhydroglucose pour les substituants de méthyle, se situe entre 0,8 à 2,2, de préférence 1,0 à 2,0, et pour les substituants d'hydroxyéthyle entre 0,05 et 1, de préférence 0,1 et 0,3.

9. Utilisation d'éthers de polysaccharide modifiés selon l'une quelconque des revendications 6 à 8, **caractérisée en ce que** sont disponibles en tant que groupes mésogènes des radicaux cholestéryle et/ou des systèmes mésogènes entièrement synthétiques, des dérivés de stérane ou des dérivés de terpène.

10. Utilisation d'éthers de polysaccaride modifiés selon l'une quelconque des revendications 6 à 9, **caractérisée en ce que** les systèmes mésogènes sont liés directement ou par l'intermédiaire d'un groupe intermédiaire à la chaîne polymère.

11. Utilisation d'éthers de polysaccaride modifiés selon l'une quelconque des revendications 6 à 10, **caractérisée en ce que** le nombre des groupes mésogènes est de 0,0001 à 0,2, de préférence de 0,001 à 0,01 par unité d'anhydroglucose.

12. Utilisation d'éthers de polysaccharide modifiés selon l'une quelconque des revendications 6 à 11, **caractérisée en ce que** les groupes mésogènes sont un radical cholestéryle lié en tant qu'ester d'acide carbonique, éventuellement dérivé.

13. Utilisation d'éthers de polysaccharide modifiés selon l'une quelconque des revendications 6 à 12, **caractérisée en ce qu'**ils contiennent en tant qu'autre radical le produit de réaction d'un agent de modification coloré.

14. Utilisation d'éthers de polysaccharide modifiés selon la revendication 13, **caractérisée en ce qu'**ils contiennent en tant qu'autre agent de modification un colorant réactif ou un produit de réaction d'un dérivé de fluorescéine réactif lié de manière covalente.

15. Utilisation d'éthers de polysaccharide modifiés selon l'une quelconque des revendications 6 à 15, **caractérisée en ce qu'**ils possèdent du fait d'un autre radical un caractère de polyélectrolyte.
